# EUROPÄISCHE PATENTSCHRIFT

(11) **EP 4 274 515 B1**
(45) Veröffentlichungstag und Bekanntmachung des Hinweises auf die Patenterteilung: **01.10.2025**
(21) Anmeldenummer: 22713518.3
(22) Anmeldetag: 07.02.2022
(51) Int. Cl.: A61F 2/28, A61F 2/30

(54) **MEHRTEILIGES IMPLANTAT MIT STÜTZELEMENT UND FUNKTIONSELEMENT**
MULTI-PART IMPLANT WITH SUPPORT ELEMENT AND FUNCTIONAL ELEMENT
IMPLANT EN PLUSIEURS PARTIES AVEC ÉLÉMENT DE SOUTIEN ET ÉLÉMENT FONCTIONNEL

(30) Priorität: 23.02.2021 DE 102021201695
(43) Veröffentlichungstag der Anmeldung: 15.11.2023
(73) Patentinhaber: Karl Leibinger Asset Management GmbH & Co. KG, 78570 Mühlheim an der Donau (DE)
(72) Erfinder: AKSU, Adem, 78054 VS-Schwenningen (DE); GROM, Stefanie, 78579 Neuhausen (DE); REINAUER, Frank, 78576 Emmingen-Liptingen (DE); WOLFRAM, Tobias, 63303 Dreieich (DE)
(74) Vertreter: Isarpatent
(86) Internationale Anmeldenummer: PCT/EP2022/052836
(87) Internationale Veröffentlichungsnummer: WO 2022/179835

(56) Entgegenhaltungen:
- WO-A1-2020/053867
- US-A1- 2005 112 397
- US-A1- 2008 147 187
- US-B2- 10 369 009

## Beschreibung

### Technisches Gebiet

Die vorliegende Erfindung betrifft ein mehrteiliges Implantat gemäß dem Oberbegriff des Anspruchs 1, wie aus der WO 2020/053867 A1 bekannt.

Insbesondere betrifft die vorliegende Erfindung Implantate für Knochen, bei denen strukturell eine gewisse Härte und Steifheit des Implantats erforderlich ist, während andererseits eine gute Resorbierbarkeit und/oder Durchdringbarkeit des Implantats für körpereigenes Gewebe gewünscht ist.

Die US 10 369 009 B2 offenbart verschiedene Wirbelsäulenimplantate mit einer Vielzahl von möglichen Einsätzen. Die Implantate werden zwischen zwei Wirbeln fixiert.

Die US 2005/112397 A1 offenbart eine spongiöse Struktur, die mehrere verbundene Platten aufweist, wobei jede Platte ein regelmäßiges Muster von massiven Bereichen, Rippen und Öffnungen aufweist, die eine transversale Abmessung und eine longitudinale Abmessung aufweisen, die nicht größer als annähernd das Vierfache der transversalen Abmessung ist, wobei mindestens eine der Öffnungen einer ersten Platte teilweise mindestens eine der Öffnungen mindestens einer anderen Platte überlappt, und wobei die massiven Bereiche aneinander ausgerichtet sind, um ein Muster von Versteifungselementen zu erzeugen.

Die US 2008/147187 A1 offenbart ein Implantat umfasssend eine metallische Stützstruktur und ein poröses metallisches Material, das durch mindestens eine Öffnung innerhalb der metallischen Stützstruktur eingebettet werden kann.

### Hintergrund der Erfindung

Knochendefekte können heutzutage mit einer Vielzahl von Methoden behandelt werden. Solche Knochendefekte treten beispielsweise bei Resektionen auf, nach Traumata, oder als Folge von Knochenentzündungen oder dergleichen. Besonders bevorzugt für die Behandlung ist üblicherweise die körpereigene, autogene Knochentransplantation. Die Verfügbarkeit von Knochenmasse für derartige Knochentransplantationen ist allerdings begrenzt, und es kann an der Entnahmestelle zu Komplikationen kommen.

Eine Alternative stellen Biomaterialien als Knochenersatzmaterial dar, welche eine osteokonduktive Wirkung aufweisen, d.h. dazu in der Lage sind, als Leitgerüst für natürliches Knochenwachstum zu fungieren. Besonders bevorzugt sind hierfür Biomaterialien, welche biokompatibel sind und nach einer Einheilungszeit durch regenerierten neuen Knochen (d.h. neues Knochenmaterial) ersetzt wurden.

Allerdings werden Implantate herkömmlicherweise mittels Fixierungselementen wie Schrauben oder Nägeln positioniert. Dies setzt eine grundlegende anfängliche Festigkeit des Implantats bzw. Implantatmaterials voraus. Bei biomechanisch schwächeren Implantatsystemen, z.B. aus resorbierbaren keramischen oder polymeren Materialien, kann die initiale Festigkeit ein Problem sein. Konventioneller Weise wird dieses durch erhöhten Materialeinsetz und größere Konstruktionen gelöst. Diese Lösung widerspricht allerdings der Zielsetzung, möglichst wenig Fremdmaterial in einen Körper einzuführen und wird durch geometrische und stereotypische Randbedingungen in ihrer Anwendbarkeit limitiert.

### Zusammenfassung der Erfindung

Es ist die Aufgabe der vorliegenden Erfindung, ein verbessertes Implantat bereitzustellen. Die Aufgabe wird durch ein Implantat mit den Merkmalen des Patentanspruchs 1 gelöst.

Dementsprechend wird ein mehrteiliges Implantat (insbesondere ein zweiteiliges Implantat) bereitgestellt, welches ein Stützelement zum Fixieren des Implantats an einem organischen Hartgewebe, insbesondere an Knochenmaterial wie etwa einem Plattenknochen oder einem Röhrenknochen, umfasst. Eine Aufnahmestruktur des Stützelements bildet (oder: formt) einen Aufnahmeraum, insbesondere derart, dass der Aufnahmeraum nach außen hin zugänglich bleibt (d.h. nicht vollständig abgeschlossen ist). Das Implantat umfasst außerdem ein in den Aufnahmeraum einbringbares (oder eingebrachtes) Funktionselement. Das Funktionselement ist durch das Stützelement (genauer: durch die Aufnahmestruktur des Stützelements) zumindest bezüglich eines Freiheitsgrades in dem Aufnahmeraum fixierbar (oder fixiert).

Bevorzugt ist die Aufnahmestruktur derart ausgebildet, dass darin das Funktionselement bezüglich mehrerer, besonders bevorzugt bezüglich aller, Freiheitsgrade fixierbar oder fixiert ist. In einigen Ausführungsformen kann vorgesehen sein, dass die vollständige Fixierung bezüglich aller Freiheitsgrade des Funktionselements erst nach Fixieren des Stützelements an dem Knochenmaterial des Patienten erfolgt.

Insbesondere die grundlegende Idee, ein Implantat (mindestens) zweiteilig auszubilden, nämlich mit einem Stützelement und einem von diesem gehaltenen und fixierten Funktionselement ermöglicht es, eine gewisse Steifheit und Robustheit des Implantats bereitzustellen, welche z.B. für das passgenaue und verschiebungsfeste Fixieren des Implantats in/an dem menschlichen Körper benötigt werden, ohne dass dadurch die Material- und Strukturwahl für einen funktionalen Teil des Implantats in beträchtlichem Maße eingeschränkt zu werden braucht. Unter dem Funktionselement (oder: funktionalen Teil) des Implantats kann derjenige Teil verstanden werden, welcher das körpereigene Gewebe, teilweise oder vollständig, durch biologische, chemische und/oder physikalische Interaktion ersetzen und/oder von diesem resorbiert werden soll.

Somit ist es eine Idee der vorliegenden Erfindung, den funktionalen Teil des Implantats durch das Funktionselement bereitzustellen, während das Stützelement die Fixierung und/oder Stabilisierung des Implantats, und hierbei insbesondere des Funktionselements, besorgt.

Das Stützelement kompensiert somit die biomechanischen Nachteile des tendenziell weicheren und/oder spröderen Funktionselements des Implantats, welches auch als interaktiver und integrativer "Biokäfig" bezeichnet werden kann. Zudem können bei der Auswahl des Materials für das Funktionselement dessen mechanische Eigenschaften weitgehend außer Acht gelassen werden, sodass ein Fokus auf das biologisch und medizinisch beste und vielversprechendste Material gelegt werden kann. Denn bei dem erfindungsgemäßen Implantat wird die mechanische Stabilität des Implantats als Ganzes nicht, oder kaum, von dem Funktionselement bereitgestellt, sondern rührt stattdessen von dem Stützelement. Das Stützelement stellt somit zusätzliche Möglichkeiten zur mechanischen Fixierung und geometrischen Anordnung sowie stereospezifische Lösungen für ansonsten nicht optimal implantierbare Biokäfige (funktionale Implantatteile) bereit. Das Stützelement kann auch als Gerüstelement bezeichnet werden.

Das Implantat kann somit insbesondere so ausgebildet sein, dass die Fixierung des Implantats in einem Hartgewebeteil des Patienten (z.B. Plattenknochen, Röhrenknochen) stattfindet, während die biologische Aktivierung des Funktionselements in anderen, bevorzugt weichgewebsartigen, Gewebestrukturen stattfinden kann. Zu den weichgewebsartigen Gewebestrukturen (oder: Weichgewebestrukturen) gehören beispielsweise Knorpel, Muskelgewebe oder Nervengewebe.

Sowohl das Stützelement als auch das Funktionselement können, je nach gewünschter Anwendung, aus resorbierbarem und/oder nicht-resorbierbaren Materialien ausgebildet sein. Sowohl das Stützelement als auch das Funktionselement können jeweils aus einer Mischung aus resorbierbaren und nicht-resorbierbaren Materialien ausgebildet sein. Bevorzugt ist es, dass das Stützelement aus nicht-resorbierbarem Material ausgebildet ist, und dass das Funktionselement zumindest teilweise, oder vollständig, aus resorbierbarem Material ausgebildet ist.

Das Funktionselement ist besonders bevorzugt porös ausgebildet, um eine Durchblutung des Funktionselements zu erleichtern. Das Stützelement hingegen ist bevorzugt massiv (d.h. nichtporös) ausgebildet, um ein etwaiges Einwachsen von Weichgewebe in das Stützelement zu verhindern. Auf diese Weise kann die Knochenregeneration auf die gewünschte Geometrie beschränkt werden.

Besonders vorteilhaft ist die Erfindung somit anwendbar, falls das ideale, d.h. medizinisch bevorzugte, Funktionselement zu spröde oder zu weich oder aus anderen Gründen nicht direkt oder nicht allein implantiert werden kann, z.B. weil es nicht mittels den üblichen Befestigungsmitteln (Schrauben, Nägeln, Klebstoffen etc.) an/in dem Patienten fixiert werden kann, oder für sich selbst die strukturelle Integrität nicht aufrechterhalten kann. Bei bevorzugten Materialkombinationen von Stützelement und Funktionselement wird somit bei den meisten Ausführungsformen das Material des Stützelements härter als das Material des Funktionselements und/oder weniger spröde als das Material des Funktionselements ausgebildet (bzw. gewählt) sein.

Das Stützelement besteht außerdem bevorzugt aus einem Material, welches keine Knochenzellen an der Hülle (Schutzhülle) aufnimmt und somit nach erfolgter Knochenregeneration leicht entfernbar ist. Somit kann bei manchen Anwendungen nach erfolgter Knochenregeneration (oder genügender Stabilität des mit dem Gewebe interagierenden Funktionselements) das Stützelement wieder aus dem Körper des menschlichen oder tierischen Patienten extrahiert werden. Bei anderen Anwendungen kann hingegen vorgesehen sein, dass das Stützelement auch dann noch in dem Körper implantiert bleibt, wenn eine (bestmögliche bzw. weitestgehende oder vollständige) Resorption des Funktionselements abgeschlossen ist. Das Verbleiben des Stützelements kann insbesondere dann sinnvoll sein, wenn dessen Außenkontur die Außenkontur eines Knochens an einer Fehlstelle ersetzt.

Die Fixierung des Stützelements an dem Knochenmaterial kann mit beliebigen Befestigungsmitteln erfolgen, beispielsweise mittels (einer oder mehrerer) Schrauben, Pins, Nägel, Drähte, mittels Nahtmaterial, und/oder mittels Kleber.

In ein und dasselbe äußere Stützelement können, je nach anatomischer Beschaffenheit und Einsatzzweck, auch mehrere Funktionselemente einsetzbar oder eingesetzt sein. Jedes Funktionselement ist bevorzugt einteilig ausgebildet, besonders bevorzugt mittels additiver Fertigung hergestellt. Alternativ kann ein Funktionselement auch mehrteilig aus mehreren Teilen zusammengesetzt sein, beispielsweise um ein Gewebeteil zwischen den beiden Teilen während der Implantierung des Implantats aufzunehmen. Hierbei kann das äußere Stützelement 20 nicht nur dazu dienen, das Funktionselement als Ganzes zu fixieren, sondern auch dazu, die beiden (oder mehreren) Einzelteile eines Funktionselements aneinander zu fixieren (und somit gegebenenfalls auch ein dazwischen eingeschlossenes Gewebeteil).

Gemäß einigen bevorzugten Ausführungsformen, Varianten oder Weiterbildungen von Ausführungsformen ist das Stützelement als ein äußeres Stützelement ausgebildet und der Aufnahmeraum ein von dem äußeren Stützelement teilweise umschlossener Innenraum. "Teilweise umschlossen" soll insbesondere bedeuten, dass der Innenraum nach außen noch teilweise offen ist, um das Funktionselement aufnehmen zu können. Bevorzugt ist der Aufnahmeraum bzw. der Innenraum nach Einbringung (oder: Einführung) des Funktionselements vollständig ausgefüllt. In manchen Ausführungsformen findet das Funktionselement vollständig in dem Innenraum Platz; in anderen Ausführungsformen steht das Funktionselement über den Innenraum hinaus.

Die Ausbildung des Stützelements als äußeres Stützelement hat den Vorteil, dass das Stützelement so beispielsweise als Schutz für das innenliegende Funktionselement (welches in diesem Fall auch als "Innenelement" bezeichenbar ist) fungieren kann, sowie das Funktionselement von außen fixieren und auch strukturell zusammenhalten kann. Vorteilhaft werden auf diese Weise auch hohe Krafteinwirkungen von außen auf das Funktionselement verhindert, sodass eine Knochenregeneration störungsfrei und ohne Verschiebung des Funktionselements fortlaufen kann.

Gemäß einigen bevorzugten Ausführungsformen, Varianten oder Weiterbildungen von Ausführungsformen weist das Stützelement eine Fensterstruktur oder eine Rippenstruktur auf, durch welche hindurch das Funktionselement zugänglich ist, insbesondere zusätzlich zu einer Öffnung, durch welche das Funktionselement in den Aufnahmeraum des Stützelements einbringbar ist. Auf diese Weise kann Gewebe schneller und einfacher mit dem Funktionselement interagieren, und eine Röntgentransparenz des Implantats kann verbessert werden. Vorzugsweise verschließt das Funktionselement, wenn es bestimmungsgemäß in das Stützelement eingefügt ist, die Fensterstruktur bzw. die Rippenstruktur. Die Fensterstruktur kann einen rundum geschlossenen Rahmen aufweisen, oder auch nur einen Teil eines rundum verlaufenden Rahmens aufweisen. Insbesondere kann eine Kante, oder ein Kantenabschnitt, von der Fensterstruktur frei gelassen werden, beispielsweise um einen Zugang zu Gewebeaufnahmestrukturen zu bieten.

In anderen Ausführungsformen kann das implantierbare Stützelement so ausgebildet sein, dass es im Inneren des Implantats positioniert ist und das Funktionselement von innen heraus fixiert und/oder strukturell stabilisiert.

Das Funktionselement kann vorzugsweise aus einem biokompatiblen, resorbierbaren Biomaterial ausgebildet sein. Das Funktionselement kann beispielsweise aus Tricalciumphosphat (β-TCP), Hydroxylapatit (HA) oder aus einem biodegradierbaren Verbundwerkstoff (aus z.B. Metall, Polymer, Keramik oder Bioglass) ausgebildet sein, welche eine knochenähnliche Zusammensetzung und gute Osteokonduktivität aufweisen. In einigen Ausführungsformen besteht das Funktionselement aus einem mit Calciumcarbonat oder Magnesium versetzten biodegradierbaren und biokompatiblen Polymer. Ebenfalls können auch spezifische Ausführungsformen mit einem Apatit versetzt sein.

Generell mögliche Materialien für das Funktionselement sind alle möglichen resorbierbaren Systeme wie etwa biodegradierbares Magnesium, biodegradierbare Magnesiumlegierungen, biodegradierbare Eisenlegierungen, biodegradierbare Zinklegierungen, biodegradierbare keramische System, bioresorbierbare Polymere oder Copolymere oder Hybridvarianten bzw. Mischungen oder Kombinationen aus den vorgenannten Materialien.

Geeignete biodegradierbare und biokompatible Polymere umfassen beispielsweise: Polyactid, z.B. PLLA (engl. "Poly(L-Lactic Acid") oder PDLLA, (engl. "Poly(D,L-Lactic Acid)") oder PGS (Polyglutaminsäure, engl. "PGA" für "Polyglutamic acid") oder PLGA (engl. "Poly(lactid-co-glycolid)", ein Copolymer aus den Monomeren Lactid und Glycolid) oder PCL (engl. Polycaprolactone).

Gemäß einigen bevorzugten Ausführungsformen, Varianten oder Weiterbildungen von Ausführungsformen ist das Stützelement aus einem nicht-resorbierbaren Material, insbesondere aus Titan oder einer Titanlegierung, aus PEEK (Polyetheretherketon), aus Implantatstahl und/oder UHMWPE (engl. "Ultra-High-Molecular-Weight Polyethylene", z.B. Markennamen Dyneema, IZANAS oder Spectra), ausgebildet.

Gemäß einigen bevorzugten Ausführungsformen, Varianten oder Weiterbildungen von Ausführungsformen ist das Funktionselement in den Aufnahmeraum einklemmbar und/oder einclipsbar (oder ist eingeklemmt bzw. eingeclipst). Hierzu kann insbesondere der Aufnahmeraum durch eine umgestülpte Kante des Stützelements gebildet sein, wobei das Funktionselement in die umgestülpte Kante (oder: Umstülpung) einklemmbar ist. Auf diese Weise kann das Funktionselement ohne Verwendung von zusätzlichen Befestigungsmitteln bezüglich des Stützelements fixiert werden, und daraufhin zusammen mit dem Stützelement z.B. an einem Knochen des Patienten fixiert werden.

Die umgestülpte Kante kann eine Krümmung aufweisen, sodass die umgestülpte Kante das Funktionselement an mehreren Seiten bzw. in mehreren Dimensionen oder Freiheitsgraden fixieren oder begrenzen kann. Es können auch mehrere umgestülpte Kanten vorhanden sein, zwischen denen das Funktionselement initial eingeklemmt wird.

Bei diesen Ausführungsformen ist es besonders vorteilhaft, wenn das Funktionselement als flache oder platte Struktur mit einer Dicke von 300-3000 Mikrometer, bevorzugt zwischen 500 und 2500 Mikrometer, besonders bevorzugt zwischen 900 und 1800 Mikrometer, ausgebildet ist. Alternativ kann das Funktionselement auch insgesamt breiter oder mit anderen Formen ausgebildet sein, jedoch an zumindest einer Kante flacher ausgebildet sein, sodass das Funktionselement mit dieser Kante in die Ausstülpung (d.h. den Aufnahmeraum) an dem Stützelement einklemmbar oder einclipsbar ist.

Gemäß einigen bevorzugten Ausführungsformen, Varianten oder Weiterbildungen von Ausführungsformen weist ein Inneres des Funktionselements mindestens eine Ausnahmestruktur auf, welche dazu ausgebildet ist, die Oberfläche des Funktionselements zu vergrößern, etwa um eine möglichst großflächige Interaktion des Funktionselements mit dem natürlichen Gewebe des Patienten zu ermöglichen.

Die mindestens eine Ausnahmestruktur kann mindestens eine (bevorzugt jeweils eine Vielzahl) Höhlung und/oder mindestens einen Tunnel und/oder mindestens eine Sackbohrung in dem Funktionselement umfassen. Die Dimensionen der mindestens einen Ausnahmestruktur können so ausgelegt sein, dass ein Kapillareffekt erzeugt oder unterbunden wird.

Unabhängig vom Vorhandensein von einer oder mehreren Ausnahmestrukturen wird außerdem bevorzugt, dass das Funktionselement porös ist, um eine bessere Durchblutung (oder Durchdringung durch andere Flüssigkeiten wie z.B. Lymphflüssigkeit etc.) zu ermöglichen. Erfindungsgemäß weist das Funktionselement eine Gewebeaufnahmestruktur auf, in welche ein Gewebeteil einführbar ist und/oder durch welches ein Gewebeteil hindurchführbar ist. Es kann eine einzelne Gewebeaufnahmestruktur mit mehreren Öffnungen nach außen vorgesehen sein, oder mehrere Gewebeaufnahmestrukturen. Vorteilhaft weist das Stützelement Öffnungen auf, welche mit der Öffnung oder den Öffnungen der Gewebeaufnahmestruktur korrespondieren, wenn das Funktionselement in das Stützelement eingesetzt ist. Solche Gewebeteile (oder Gewebestrukturen) können beispielsweise Blutgefäße (Adern, Venen, Kapillargefäße...) oder Membranen sein. Gewebeaufnahmestrukturen können als nach außen offene Gräben oder Kanäle ausgebildet sein, etwa um die Einführung oder das Einbetten von Gewebeteilen zu erleichtern. Gewebeaufnahmestrukturen können jedoch beispielsweise auch unilateral verschlossen sein, beispielsweise wenn ein gerichtetes Gewebeeinwachsen erzielt werden soll. Allgemein können verschiedenseitig offene und geschlossene Gewebeaufnahmestrukturen vorgesehen werden, je nachdem, was für ein Gewebeteil eingesetzt werden oder einwachsen soll, von welcher Seite und bis wo hin dies erfolgen soll und dergleichen Überlegungen mehr.

Ein nicht erfindungsgemäßes Verfahren zum Implantieren eines erfindungsgemäßen Implantats, umfasst die Schritte:
Einlegen (insbesondere Einklemmen oder Einclipsen) des Funktionselements in den Aufnahmeraum des Stützelements; Einbringen des Stützelements mit in dem Aufnahmeraum eingelegtem Funktionselement in einen menschlichen oder tierischen Körper eines Patienten; Fixieren des Stützelements an einem Knochen zum Fixieren des Implantats. Ein Schritt ist ein Einbringen eines Gewebeteils, oder einer Gewebestruktur, des Patienten in eine Gewebeaufnahmestruktur des Implantats, wobei dieses Einbringen des Gewebeteils vor, während und/oder nach dem Einbringen des äußeren Stützelements in den Patienten durchgeführt werden kann. Ein weiterer optionaler Schritt kann das Entfernen des Stützelements aus dem Körper des Patienten ohne Entfernen des Funktionselements aus dem Körper des Patienten umfassen.

### Kurze Beschreibung der Figuren

Die Erfindung wird nachstehend anhand von Ausführungsbeispielen in den Figuren der Zeichnung näher erläutert. In teilweise schematisierter Darstellung zeigen hierbei:
- Fig. 1: eine schematische Übersicht über eine Anwendung von Implantaten gemäß einer ersten und gemäß einer zweiten Ausführungsform;
- Fig. 2: eine zusammengesetzte Ansicht des Implantats aus Fig. 1 gemäß der ersten Ausführungsform von der Seite aus gesehen;
- Fig. 3: eine Querschnittsansicht durch das Implantat aus Fig. 2;
- Fig. 4: eine Ansicht des Implantats aus Fig. 1 gemäß der zweiten Ausführungsform;
- Fig. 5: eine schematische Darstellung einer Draufsicht von oben auf ein Implantat gemäß einer dritten Ausführungsform der vorliegenden Erfindung;
- Fig. 6a: eine schematische Seitenansicht des Implantats aus Fig. 5;
- Fig. 6b: eine schematische Querschnittsansicht des Implantats auf Fig. 5.

In sämtlichen Figuren sind gleiche bzw. funktionsgleiche Elemente und Vorrichtungen - sofern nichts anderes angegeben ist - mit denselben Bezugszeichen versehen worden.

### Detaillierte Beschreibung der Figuren

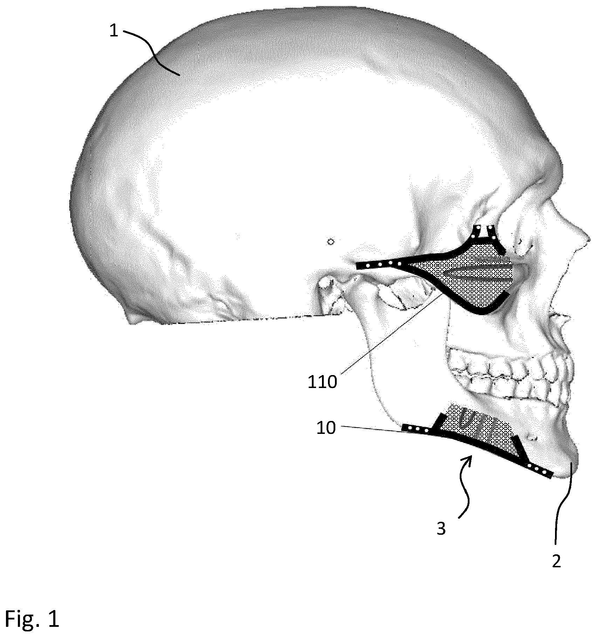

Fig. 1 zeigt eine schematische Darstellung der Anwendung eines Implantats 10 gemäß einer ersten Ausführungsform der vorliegenden Erfindung sowie eines Implantats 110 gemäß einer zweiten Ausführungsform der vorliegenden Erfindung.

Dargestellt ist in Fig. 1 außerdem ein menschlicher Schädel 1. Für die Beschreibung des Implantats 10 wird angenommen, dass an einem Unterkiefer 2 dieses Schädels ein Stück 3 fehlt, z.B. aufgrund eines Unfalls oder einer vorgenommenen Resektion. Das heißt, im vorliegenden Fall wird das Implantat 10 für einen Plattenknochen eingesetzt. Für das Implantat 110 wird eine Fehlstelle am rechten Wangenknochen des Schädels 1 angenommen.

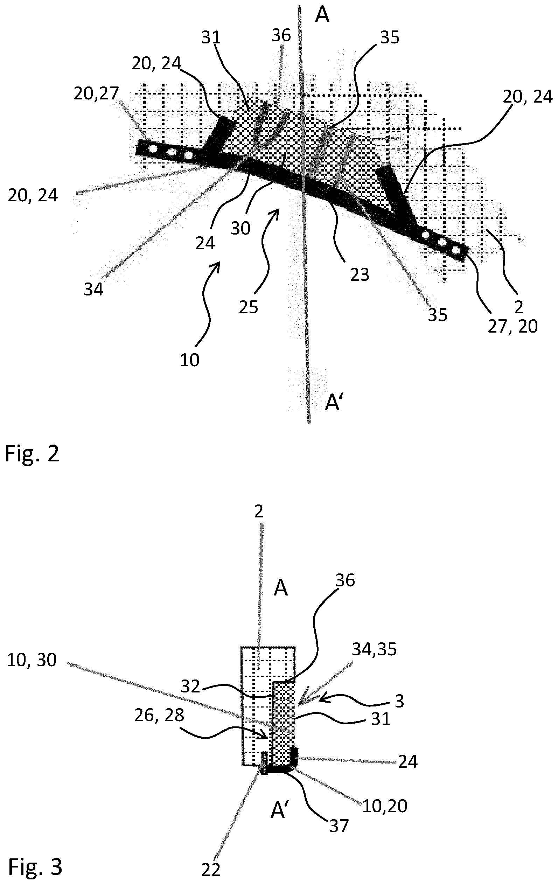

Das Implantat 10 dient dazu, die Fehlstelle 3 im Unterkiefer 2 zumindest auf kurze Sicht durch künstliches Material zu ersetzen. Hierzu umfasst das Implantat 10 ein äußeres Stützelement sowie ein resorbierbares Funktionselement, die im Folgenden auch mit Bezug auf die Fig. 2 und Fig. 3 näher erläutert werden. Das Funktionselement soll hierbei die natürliche Knochenregeneration fördern und leiten und mittelfristig in regeneriertem Knochengewebe resorbiert werden.

Fig. 2 zeigt eine Ansicht des Implantats 10 aus Fig. 1 im implantierten Zustand von einer Außenseite aus gesehen, wobei mit Außenseite in diesem Fall diejenige Seite gemeint ist, welche von dem Schädel 1 (oder allgemein: von dem zu behandelnden, zu ersetzenden oder zu ergänzenden Knochen) abgewandt ist, wenn das Implantat 10 bestimmungsgemäß implantiert wurde. In Fig. 2 ist zu sehen, wie das Funktionselement 30 in das äußere Stützelement 20 eingesetzt und mit dieser zusammen bereits implantiert, d.h. insbesondere an dem Knochen (hier Unterkiefer 2) fixiert, ist.

Das Stützelement 20 kann beispielsweise aus einem nicht-resorbierbaren Material, insbesondere aus Titan oder einer Titanlegierung, aus PEEK (Polyetheretherketon), aus Implantatstahl und/oder UHMWPE (engl. "Ultra-High-Molecular-Weight Polyethylene", z.B. Markennamen Dyneema, IZANAS oder Spectra), ausgebildet sein.

Das äußere Stützelement 20 ist mit einer langgestreckten Trägerstruktur 23 ausgebildet, an deren beiden longitudinalen Enden jeweils Befestigungsabschnitte angeordnet sind, in oder an denen Befestigungsmittel, oder Hilfsmittel für Befestigungsmittel, vorliegend jeweils drei Schraublöcher 27, ausgebildet sind. Außer Schraublöchern 27 können selbstverständlich auch andere Befestigungsmittel, oder Hilfsstrukturen für Befestigungsmittel (wie etwa Schraublöcher für Schrauben) an dem äußeren Stützelement 20 ausgebildet sein. Möglich wären auch Lochstrukturen für die Aufnahme von Pin-Systemen, die zur temporären Fixierung dienen können.

Zwischen den Befestigungsabschnitten der Trägerstruktur 23 ist als weiterer Teil des äußeren Stützelements 20 eine Fensterstruktur 25 ausgebildet, durch welche das Funktionselement 30 (genauer: eine Außenseite 31 des Funktionselements 30) im nicht implantierten Zustand zu sehen und zugänglich ist. Somit kann organisches Gewebe, welches das Implantat 10 nach Implantation an der Außenseite des äußeren Stützelements 20 bedeckt, direkt in Kontakt mit der Außenseite 31 des Funktionselements 30 treten. Darüber hinaus kann eine solche Fensterstruktur 25 eine größtmögliche Röntgentransparenz des Implantats 10 ermöglichen.

Der Abschnitt des äußeren Stützelements 20 um die Fensterstruktur 25 herum kann als Rahmenstruktur 24 bezeichnet werden. Vorliegend ist die Rahmenstruktur 24 nicht völlig um die Fensterstruktur 25 herum geschlossen, insbesondere umschließt sie das in der Seitenansicht viereckige Funktionselement 30 nur an einer Kante vollständig (durch einen Teil der Trägerstruktur 23) sowie an zwei weiteren Kanten lediglich teilweise, bevorzugt mindestens zur Hälfte. Der jeweils andere Teil dieser zwei weiteren Kanten kann beispielsweise durch Knochenmaterial umschlossen bzw. berührt sein, wie anhand von Fig. 3 noch deutlicher werden wird. An der vierten Kante 36 ist das Funktionselement 30 bei der gezeigten Ausführungsform durch den Unterkieferknochen 3 selbst begrenzt, wie mit Bezug auf Fig. 3 im Folgenden noch genauer beschrieben werden wird.

Die Rahmenstruktur 24 umfasst das Funktionselement 30 außerdem auch an seiner Außenseite 31 zumindest teilweise, um es in dieser Richtung zu fixieren, wie anhand von Fig. 3 noch genauer beschrieben werden wird.

Das resorbierbare Funktionselement 30 kann beispielsweise aus biodegradierbarem Magnesium, aus einer biodegradierbaren Magnesiumlegierung, aus einer biodegradierbaren Eisenlegierung, aus einer biodegradierbaren Zinklegierung, aus einem biodegradierbaren keramischen System, aus einem bioresorbierbaren Polymere oder Copolymer oder Hybridvarianten bzw. Mischungen oder Kombinationen aus den vorgenannten Materialien ausgebildet sein.

Das Funktionselement 30 kann im Inneren und/oder an der Oberfläche des Funktionselements 30 ausgebildete Ausnahmestrukturen aufweisen. Bei den Ausnahmestrukturen kann es sich insbesondere um mindestens eine (bevorzugt jeweils eine Vielzahl) Höhlung und/oder mindestens einen Tunnel und/oder mindestens eine Sackbohrung in dem Funktionselement 30 handeln. Die Dimensionen der mindestens einen Ausnahmestruktur können so ausgelegt sein, dass ein Kapillareffekt erzeugt oder unterbunden wird.

Die vorteilhafte Herstellung des Funktionselements 30 mittels additiver Fertigung ermöglicht es, plangenau und äußert präzise diese Ausnahmestrukturen auszubilden.

Fig. 2 verdeutlicht außerdem, dass das Funktionselement 30 eine erste Gewebeaufnahmestruktur 34 sowie zwei weitere Gewebeaufnahmestrukturen 35 aufweist.

Diese kann, wie im vorliegenden Fall, beispielsweise als durchgehender Graben, insbesondere in der Außenseite 31 des Funktionselements 30, oder als durchgehender Tunnel ausgebildet sein. Die erste Gewebeaufnahmestruktur 34 ist als im vorliegenden Beispiel als durchgehender U-förmiger Graben ausgebildet, dessen longitudinale Enden beide an derselben vierten Kante 36 des Funktionselements 30 angeordnet sind. Die weiteren Gewebeaufnahmestrukturen 35 sind als lineare, gerade Gräben ausgebildet, welche sich von der vierten Kante 36 des Funktionselements 30 bis hin zu der gegenüberliegenden Kante des Funktionselements 30 erstrecken, welche an dem äußeren Stützelement 20 (genauer: an der Rahmenstruktur 24 und der Trägerstruktur 23) anliegt. Jede Gewebeaufnahmestruktur 34, 35 kann beispielsweise auch unilateral verschlossen sein, beispielsweise wenn ein gerichtetes Gewebeeinwachsen erzielt werden soll.

In die Gewebeaufnahmestrukturen 34, 35 kann nach, oder während, der Implantation des Implantats ein Gewebeteil, oder eine Gewebestruktur, des Patienten eingebracht werden, insbesondere von der Außenseite 31 und/oder der Kante 36 des Funktionselements 30 her. Auf diese Weise können somit gewünschte Verläufe dieses Gewebeteils bezüglich des Implantats 10 vorteilhaft vorgegeben werden. Bei dem Gewebeteil kann es sich beispielsweise um ein Blutgefäß oder um eine Membran oder dergleichen handeln.

Es versteht sich, dass bei anderen Einsatzorten, oder anderen anatomischen Gegebenheiten, die Gewebeaufnahmestrukturen 34, 35 auch als Sackbohrung mit nur einer einzelnen Öffnung ausgebildet sein können, dass die Öffnung (oder die Öffnungen) nicht an einer Kante angeordnet sein müssen, dass sich Ein- und Ausgangsöffnung an verschiedenen Kanten befinden können und dergleichen mehr. Diese Betrachtungen gelten für platte, flache Funktionselemente 30 wie in dem vorliegenden Beispiel. Es versteht sich, dass bei anderen geometrischen Formen des Funktionselements 30 noch viele weitere Möglichkeiten der Ausgestaltung und Anordnung von Öffnungen der Gewebeaufnahmestrukturen 34, 35 möglich sind.

Fig. 3 zeigt eine schematische Querschnittsansicht durch das Implantat 10 aus Fig. 2 entlang der Linie A-A'. In Fig. 3 ist gut erkennbar, dass das Implantat 10 im vorliegenden Fall als "onlay" ausgeführt ist, d.h., dass das Funktionselement 30 auf einem Teil des Knochens (hier: Unterkiefers 2) aufliegt. In dem Querschnitt in Fig. 3 ist sichtbar, dass das Funktionselement 30 hierbei die Fehlstelle 3 ausfüllt. In anderen Ausführungsformen oder Anwendungen kann das Funktionselement 30 auch als Komplettaugmentation ausgebildet sein.

Fig. 3 zeigt außerdem, wie die Rahmenstruktur 24 im Querschnitt gebogen ausgeführt sein kann, um das Funktionselement 30 an dessen Kante 37, welche die Rahmenstruktur 24 vollständig bedeckt (als "Außenkante" bezeichenbar), zusätzlich auch teilweise an der Außenseite 31 des Funktionselements 30 zu umfassen. Eine der Außenseite 31 gegenüberliegende Innenseite 32 des Funktionselements 30 ist vollständig von dem Unterkieferknochen 2 bedeckt, und umgekehrt. Diese Biegung der Rahmenstruktur 24 im Querschnitt bildet somit eine Aufnahmestruktur 28, welche einen Aufnahmeraum 26 für das Funktionselement 30 definiert, wobei der Aufnahmeraum 26 durch das Funktionselement 30 vollständig ausgefüllt wird, aber das Funktionselement 30 (sogar zum größten Teil) aus dem Aufnahmeraum 26 herausragt.

Die Rahmenstruktur 24 kann auch an den beiden anderen weiteren Kanten des Funktionselements 30, welche nur teilweise von der Rahmenstruktur 24 umfasst werden, im Querschnitt gebogen ausgeführt sein, um jeweils zum Teil auch noch die Außenseite 31 des Funktionselements 30 zu umfassen.

An der Kante 37 des Funktionselements 30 kann an der Innenseite 32 des Funktionselements ein Arretierungsmittel 22 angeordnet sein, oder eine Mehrzahl solcher Arretierungsmittel. Durch solche Arretierungsmittel 22, beispielsweise einen durch eine Öffnung in dem äußeren Stützelement 20 in den Knochen 2 hinein getriebenen Stift, kann eine Verbindung (oder Fixierung) des äußeren Stützelements 20 auch im Bereich zwischen den Befestigungsabschnitten an den longitudinalen Enden der Trägerstruktur 23 mit (bzw. an) dem Knochen 2 verbessert werden.

Der Aufnahmeraum 26 i dient zur Aufnahme des Funktionselements 30. Konturen des Aufnahmeraums 26 sind genau an die anliegenden Kanten des Funktionselements 30 angepasst. Bevorzugt ist der Aufnahmeraum 26 nach Aufnahme des Funktionselements 30 vollständig ausgefüllt.

Die Aufnahmetasche 28 kann eine (oder mehrere) Unterbrechung aufweisen, welche den Zugang von außerhalb des Implantats 10 auf eine Öffnung mindestens einer der Gewebeaufnahmestrukturen 34, 35 freigibt. Auch diese Unterbrechungen können jeweils dort angeordnet sein, wo gemäß einem gewünschten Einsatzort bzw. der dortigen Anatomie das Funktionselement 30 Öffnungen aufweist.

Die Aufnahmestruktur 28 ist in der gezeigten Ausführungsform durch Umstülpen der Rahmenstruktur 24 (genauer gesagt: durch Umstülpen der Kante der Rahmenstruktur 24) des äußeren Stützelements 20 in Richtung der Innenseite (d.h. in Richtung des Knochens 2) gebildet, wobei am Ende der Umstülpung in dem Querschnitt der Rahmenstruktur 24 im Wesentlichen eine 90-Grad-Biegung vorliegt. Dies ist vorliegend vorteilhaft, da bei der onlay-Anwendung hier der gesunde Unterkieferknochen 2 den Aufnahmeraum 26 an der Innenseite 32 des Funktionselements 30 abschließt (oder: bildet). Bei Anwendungen, bei denen eine Fehlstelle einer Knochenplatte vollständig durch das Funktionselement 30 ersetzt (oder: gefüllt) sein soll, kann die Umstülpung auch derart ausgebildet sein, dass sie an der Außenseite 31 des Funktionselements 30 beginnt und dieses teilweise hintergreift, d.h. mit einem flach an einer Innenseite 32 des Funktionselements 30 anliegenden Abschnitt endet.

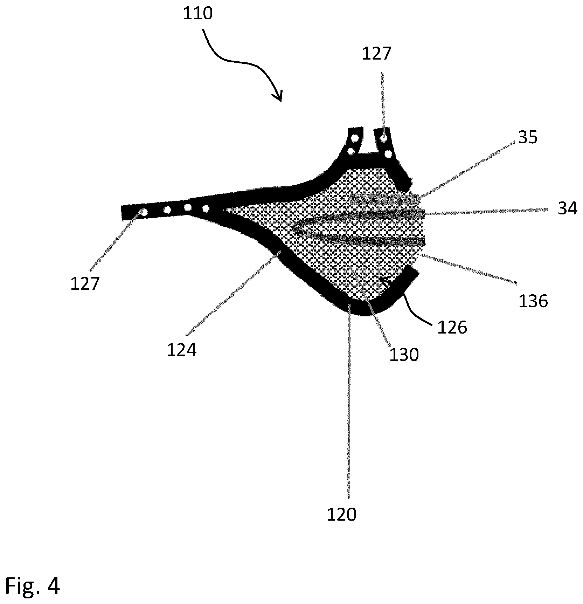

Fig. 4 zeigt eine Ansicht des Implantats 110 aus Fig. 1 gemäß der zweiten Ausführungsform. Das Implantat 110 ist eine Variante des Implantats 10 und ist ebenfalls für einen Plattenknochen ausgelegt, wie in Fig. 1 ersichtlich ist. Bei dem Implantat 110 umfasst ein äußeres Stützelement 120 wiederum eine Rahmenstruktur 124, welche einen Aufnahmeraum 126 definiert, in den ein resorbierbares Funktionselement 130 einführbar oder eingeführt ist.

Wie mit Bezug auf Fig. 3 beschrieben wurde, kann die Rahmenstruktur 124 jeweils in Richtung des Knochens umgestülpt sein, um das Funktionselement 130 lateral zu fixieren, oder sogar so weit umgestülpt sein, um das Funktionselement 130 an dessen Innenseite zu hintergreifen. Auf diese Weise bilden die Umstülpungen der Rahmenstruktur 124 eine Aufnahmestruktur 128, welche wiederum einen Aufnahmeraum 126 für das Funktionselement 130 definiert. In diesem Fall wird der Aufnahmeraum 126 wiederum vollständig durch das Funktionselement 130 ausgefüllt, wobei dieses nur zu einem sehr geringen Teil (nämlich an dem freiliegenden Kantenabschnitt 136) aus dem Aufnahmeraum 126 herausragt, oder optional auch gar nicht herausragt. Je nach Art, Lage und Grad der Umstülpung fixiert die Aufnahmestruktur 128 das Funktionselement 130 in verschiedenen Richtungen bzw. bezüglich verschiedener Freiheitsgrade.

Das äußere Stützelement 120 umfasst das Funktionselement 130 an dessen Außenseite (d.h. der vom Knochen abgewandten Seite) nur teilweise, sodass das Funktionselement 130 durch das äußere Stützelement 120 an der Außenseite zugänglich bleibt. Mit anderen Worten bildet die Rahmenstruktur 124 wiederum eine Fensterstruktur, durch welche das Funktionselement 130 für an dem Implantat 110 anliegendes Gewebe zugänglich bleibt.

Die Rahmenstruktur 124 umfasst das Funktionselement 130 fast vollständig, wobei eine Kantenabschnitt 136 des im Wesentlichen flach ausgebildeten Funktionselements 130 wiederum frei bleibt. An diesem freien Kantenabschnitt 136 können Öffnungen von Gewebeaufnahmestrukturen 34, 35 ausgebildet sein, wie dies beispielsweise mit Bezug auf Fig. 2 und Fig. 3 bereits beschrieben wurde. Angepasst an die Knochengeometrie, für welche das Implantat 110 ausgelegt ist, sind an Abschnitten des äußeren Stützelements 120 Schraublöcher 127 zum Fixieren des Stützelements 120 angeordnet.

Das äußere Stützelement 120, das Funktionselement 130 sowie die Befestigungsmittel können jeweils so gewählt werden wie im Vorangehenden beschrieben. Insbesondere kann das Stützelement 120 kann beispielsweise aus einem nicht-resorbierbaren Material, insbesondere aus Titan oder einer Titanlegierung, aus PEEK (Polyetheretherketon), aus Implantatstahl und/oder UHMWPE (engl. "Ultra-High-Molecular-Weight Polyethylene", z.B. Markennamen Dyneema, IZANAS oder Spectra), ausgebildet sein. Das resorbierbare Funktionselement 130 kann beispielsweise aus biodegradierbarem Magnesium, aus einer biodegradierbaren Magnesiumlegierung, aus einer biodegradierbaren Eisenlegierung, aus einer biodegradierbaren Zinklegierung, aus einem biodegradierbaren keramischen System, aus einem bioresorbierbaren Polymere oder Copolymer oder Hybridvarianten bzw. Mischungen oder Kombinationen aus den vorgenannten Materialien ausgebildet sein.

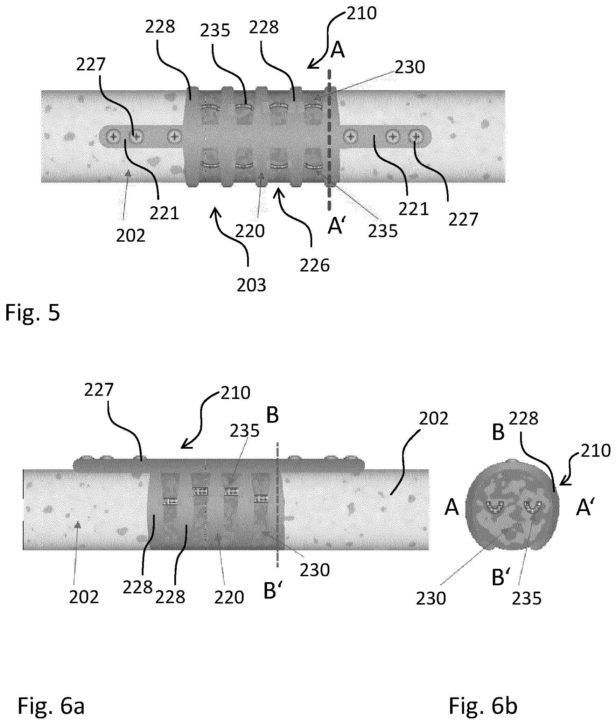

Fig. 5 zeigt eine schematische Darstellung einer Draufsicht von oben auf ein Implantat 210 gemäß einer dritten Ausführungsform der vorliegenden Erfindung. Das Implantat 210 dient dazu, eine Fehlstelle 203 in einem Röhrenknochen 202 zu beheben, d.h. auszufüllen. Dabei kommt es häufig vor, dass Material, welches sich hierfür gut eignet, etwa aufgrund seiner Resorptionseigenschaften und dergleichen, nur schwierig an dem restlichen Röhrenknochen 202 befestigt werden kann, dort aber fixiert sein muss, bis die Heilung abgeschlossen ist und/oder die Resorption vollständig durchgeführt wurde.

Fig. 6a zeigt dieselbe Situation wie in Fig. 5, jedoch aus einer Seitenansicht; Fig. 6b zeigt eine Querschnittsansicht entlang des Schnitts A-A` in Fig. 5 bzw. entlang des Schnitts B-B' in Fig. 6a.

Das Implantat 210 aus Fig. 5 umfasst ein äußeres Stützelement 220 sowie ein Funktionselement 230. Das äußere Stützelement 220 umfasst einen länglichen geraden Steg 221 (oder: eine länglicher Trägerstruktur), welcher an beiden Enden Verbindungsmittel, hier Schraublöcher 227, aufweist. Mittels durch die Schraublöcher 227 eingeführten Schrauben sind die beiden Enden des Stegs 221 an jeweils einem Teil des Röhrenknochen 202 fixierbar. In dem Bereich der Fehlstelle 203 sind an dem Steg 221 zehn Rippenstrukturen 228 des äußeren Stützelements 220 angeordnet, wobei der Steg 221 bezüglich der Rippenstrukturen 128 eine Position ähnlich der menschlichen Wirbelsäule bezüglich der menschlichen Rippen annimmt. Die Rippenstrukturen 228 umschließen (oder: definieren) gemeinsam einen zylinderförmigen Innenraum 226 (oder: Aufnahmeraum).

Das Funktionselement 230 ist ebenfalls im Wesentlichen, d.h. was seine äußere Kontur angeht, zylinderförmig ausgebildet und zwar derart, dass es genau in den zylinderförmigen Innenraum 226 einbringbar ist. In axialer Richtung wird die Bewegung des Funktionselements 230 durch die beiden Teile des Röhrenknochens 202 beschränkt bzw. verhindert. In tangentialer und radialer Richtung wird die Bewegung des Funktionselements 230 durch die Rippenstrukturen 228 beschränkt bzw. verhindert. Somit bilden die Rippenstrukturen 228 eine Aufnahmestruktur für das Funktionselement 230.

Es versteht sich, dass in der Natur Röhrenknochen nicht vollständig zylinderförmig ausgebildet sind; dementsprechend versteht es sich, dass das Implantat 210 entsprechend der tatsächlichen Form des Röhrenknochens 202 angepasst sein kann.

Wie aus Fig. 6a besonders gut ersichtlich ist, kann das Implantat 210 mit in den Innenraum 226 eingesetztem Funktionselement 230 sehr gut von oben (in Fig. 6a) implantiert werden, und zwar so, dass der Innenraum 226 mit dem Funktionselement 230 genau die Fehlstelle 203 ausfüllt. Danach können Knochenschrauben in die Schraublöcher 227 eingeführt werden und das Implantat 210 somit einseitig mit dem Röhrenknochen 202 verschraubt, d.h. an diesem fixiert, werden.

Länge des Stegs 221, Anzahl der Schraublöcher 227, Anzahl der Rippenstrukturen 228 und dergleichen können jeweils an den Einsatzort angepasst werden.

Fig. 5, Fig. 6a und Fig. 6b illustrieren ebenfalls, dass das Funktionselement 230 wiederum mit Öffnungen 235 ausgebildet sein kann, welche eine Gewebeaufnahmestruktur (nicht gezeigt) im Inneren des Funktionselements 230 zugänglich machen.

Obwohl die vorliegende Erfindung anhand bevorzugter Ausführungsbeispiele vorstehend beschrieben wurde, ist sie darauf nicht beschränkt, sondern auf vielfältige Art und Weise modifizierbar. Insbesondere lässt sich die Erfindung in mannigfaltiger Weise verändern oder modifizieren, ohne vom Kern der Erfindung abzuweichen.

### Bezugszeichenliste

- 1: Schädel
- 2: Unterkiefer
- 3: Fehlstelle
- 10: Implantat
- 20: äußeres Stützelement
- 22: Arretiermittel
- 23: Trägerstruktur
- 24: Rahmenstruktur
- 25: Fensterstruktur
- 26: Innenraum
- 27: Schraublöcher
- 28: Aufnahmestruktur

- 30: Funktionselement
- 31: Außenseite des Funktionselements
- 34: Gewebeaufnahmestruktur
- 35: Gewebeaufnahmestruktur
- 36: Kante des Funktionselements
- 37: Kante des Funktionselements
- 202: Röhrenknochen
- 110: Implantat
- 124: Rahmenstruktur
- 126: Aufnahmeraum
- 127: Schraublöcher
- 128: Aufnahmestruktur
- 130: Funktionselement
- 136: Kantenabschnitt
- 203: Fehlstelle210 Implantat
- 220: äußeres Stützelement
- 221: Steg
- 226: Aufnahmeraum
- 227: Schraublöcher
- 228: Rippenstrukturen
- 230: Funktionselement
- 235: Öffnungen

## Patentansprüche

1. Mehrteiliges Implantat (10; 110; 210), umfassend:
ein Stützelement (20; 120; 220) zum Fixieren des Implantats (10; 110; 210) an einem Knochenmaterial (2; 202);
wobei eine Aufnahmestruktur (28; 128; 228) des Stützelements (20; 120; 220) einen Aufnahmeraum (26; 126; 226) bildet; und
ein in den Aufnahmeraum (26; 126; 226) einbringbares Funktionselement (30; 130; 230);
wobei das Funktionselement (30; 130; 230) durch die Aufnahmestruktur (28; 128; 228) zumindest bezüglich eines Freiheitsgrades in dem Aufnahmeraum (26; 126; 226) fixierbar ist;
**dadurch gekennzeichnet, dass**
das Funktionselement (30; 130; 230) eine Gewebeaufnahmestruktur (34, 35) aufweist, in welche ein Gewebeteil einführbar ist und/oder durch welche ein Gewebeteil hindurchführbar ist.

2. Implantat (10; 110; 210) nach Patentanspruch 1,
wobei das das Stützelement (20; 120; 220) als ein äußeres Stützelement ausgebildet ist und der Aufnahmeraum (26; 126; 226) ein von der Aufnahmestruktur (28; 128; 228) teilweise umschlossener Innenraum ist.

3. Implantat (10; 110; 210) nach Patentanspruch 2,
wobei das Stützelement (20; 120; 220) eine Fensterstruktur (25; 125) oder eine Rippenstruktur (228) aufweist, durch welche hindurch das Funktionselement (30; 130; 230) in dem Aufnahmeraum (26; 126; 226) zugänglich ist.

4. Implantat (10; 110; 210) nach einem der Patentansprüche 1 bis 3,
wobei das Funktionselement (30; 130; 230) ausgebildet ist, oder besteht, aus mindestens einem der folgenden Materialien:
- biodegradierbares Magnesium;
- biodegradierbare Magnesiumlegierung;
- biodegradierbare Eisenlegierung;
- biodegradierbare Zinklegierung;
- biodegradierbares keramisches System;
- bioresorbierbares Polymer oder Copolymer.

5. Implantat (10; 110; 210) gemäß einem der Patentansprüche 1 bis 3,
wobei das Stützelement (20; 120; 220) aus einem nicht-resorbierbaren Material, insbesondere aus Titan oder einer Titanlegierung, aus Polyetheretherketon, aus Implantatstahl und/oder aus UHMWPE ausgebildet ist.

6. Implantat (10; 110; 210) nach einem der Patentansprüche 1 bis 5,
wobei das Funktionselement (30; 130; 230) in den Aufnahmeraum (26; 126; 226) einklemmbar und/oder einclipsbar ist.

7. Implantat (10; 110) nach einem der Patentansprüche 1 bis 6, wobei der Aufnahmeraum (26; 126) zumindest durch eine umgestülpte Kante des Stützelements (20; 120) gebildet ist.

8. Implantat (10; 110; 210) nach einem der Patentansprüche 1 bis 7,
wobei ein Inneres des Funktionselements (30; 130; 230) mindestens eine Ausnahmestruktur aufweist, welche dazu ausgebildet ist, die Oberfläche des Funktionselements (30; 130; 230) zu vergrößern.

9. Implantat (10; 110; 210) nach Patentanspruch 8,
wobei die mindestens eine Ausnahmestruktur mindestens eine Höhlung und/oder mindestens einen Tunnel und/oder mindestens eine Sackbohrung in dem Funktionselement (30; 130; 230) umfasst.

## Claims

1. Multi-part implant (10; 110; 210), comprising:
a support element (20; 120; 220) for fixing the implant (10; 110; 210) to a bone material (2; 202);
where a receiving structure (28; 128; 228) of the support element (20; 120; 220) forms a receiving space (26; 126; 226); and
a functional element (30; 130; 230) which can be introduced into the receiving space (26; 126; 226);
the functional element (30; 130; 230) being fixable in the receiving space (26; 126; 226) by the receiving structure (28; 128; 228) at least in terms of one degree of freedom;
**characterised in that**
the functional element (30; 130; 230) has a tissue receiving structure (34, 35) into which a tissue part can be inserted and/or through which a tissue part can be passed.

2. Implant (10; 110; 210) according to claim 1,
wherein the support element (20; 120; 220) is formed as an outer support element and the receiving space (26; 126; 226) is an interior space partially enclosed by the receiving structure (28; 128; 228).

3. Implant (10; 110; 210) according to claim 2,
wherein the support element (20; 120; 220) has a window structure (25; 125) or a rib structure (228) through which the functional element (30; 130; 230) in the receiving space (26; 126; 226) is accessible.

4. Implant (10; 110; 210) according to any of claims 1 to 3,
wherein the functional element (30; 130; 230) is formed from or consists of at least one of the following materials:
- biodegradable magnesium;
- biodegradable magnesium alloy;
- biodegradable iron alloy;
- biodegradable zinc alloy;
- biodegradable ceramic system;
- bioresorbable polymer or copolymer.

5. Implant (10; 110; 210) according to any of claims 1 to 3,
wherein the support element (20; 120; 220) is formed from a non-resorbable material, in particular from titanium or a titanium alloy, from polyetheretherketone, from implant steel and/or from UHMWPE.

6. Implant (10; 110; 210) according to any of claims 1 to 5,
wherein the functional element (30; 130; 230) can be clamped and/or clipped into the receiving space (26; 126; 226).

7. Implant (10; 110) according to any of claims 1 to 6,
wherein the receiving space (26; 126) is formed at least by an turned-back edge of the support element (20; 120).

8. Implant (10; 110; 210) according to any of claims 1 to 7,
wherein an interior of the functional element (30; 130; 230) has at least one recess structure configured to enlarge the surface of the functional element (30; 130; 230).

9. Implant (10; 110; 210) according to claim 8,
wherein the at least one recess structure comprises at least one cavity and/or at least one tunnel and/or at least one blind hole in the functional element (30; 130; 230).

## Revendications

1. Implant en plusieurs parties (10 ; 110 ; 210), comprenant :
un élément de soutien (20 ; 120 ; 220) destiné à fixer l'implant (10 ; 110 ; 210) sur une matière osseuse (2 ; 202) ;
une structure de réception (28 ; 128 ; 228) de l'élément de soutien (20 ; 120 ; 220) formant un espace de réception (26 ; 126 ; 226) ; et
un élément fonctionnel (30 ; 130 ; 230) qui peut être placé dans l'espace de réception (26 ; 126 ; 226) ;
l'élément fonctionnel (30 ; 130 ; 230) pouvant être fixé dans l'espace de réception (26 ; 126 ; 226) par la structure de réception (28 ; 128 ; 228) au moins par rapport à un degré de liberté ;
**caractérisé en ce que**
l'élément fonctionnel (30 ; 130 ; 230) présente une structure de réception de tissu (34, 35) dans laquelle une partie de tissu peut être introduite et/ou à travers laquelle une partie de tissu peut passer.

2. Implant (10 ; 110 ; 210) selon la revendication 1,
dans lequel l'élément de soutien (20 ; 120 ; 220) est réalisé sous la forme d'un élément de soutien extérieur et l'espace de réception (26 ; 126 ; 226) est un espace intérieur en partie entouré par la structure de réception (28 ; 128 ; 228).

3. Implant (10 ; 110 ; 210) selon la revendication 2,
dans lequel l'élément de soutien (20 ; 120 ; 220) présente une structure de fenêtres (25 ; 125) ou une structure de nervures (228) à travers laquelle l'élément fonctionnel (30 ; 130 ; 230) est accessible dans l'espace de réception (26 ; 126 ; 226).

4. Implant (10 ; 110 ; 210) selon l'une des revendications 1 à 3,
dans lequel l'élément fonctionnel (30 ; 130 ; 230) est constitué ou se compose d'au moins un des matériaux suivants :
- magnésium biodégradable ;
- alliage de magnésium biodégradable ;
- alliage de fer biodégradable ;
- alliage de zinc biodégradable ;
- système céramique biodégradable ;
- polymère ou copolymère biorésorbable.

5. Implant (10 ; 110 ; 210) selon l'une des revendications 1 à 3,
dans lequel l'élément de soutien (20 ; 120 ; 220) est constitué d'un matériau non résorbable, notamment de titane ou d'un alliage de titane, de polyétheréthercétone, d'acier pour implants et/ou de UHMWPE.

6. Implant (10 ; 110 ; 210) selon l'une des revendications 1 à 5,
dans lequel l'élément fonctionnel (30 ; 130 ; 230) peut être pincé et/ou clipsé dans l'espace de réception (26 ; 126 ; 226).

7. Implant (10 ; 110) selon l'une des revendications 1 à 6,
dans lequel l'espace de réception (26 ; 126) est formé au moins par un bord retourné de l'élément de soutien (20 ; 120).

8. Implant (10 ; 110 ; 210) selon l'une des revendications 1 à 7,
dans lequel l'intérieur de l'élément fonctionnel (30 ; 130 ; 230) présente au moins une structure d'évidement qui est conçue pour agrandir la surface de l'élément fonctionnel (30 ; 130 ; 230).

9. Implant (10 ; 110 ; 210) selon la revendication 8,
dans lequel l'au moins une structure d'évidement comprend au moins un creux et/ou au moins un tunnel et/ou au moins un trou borgne dans l'élément fonctionnel (30 ; 130 ; 230).
